(19) **Europäisches Patentamt European Patent Office Office européen des brevets**

(11) **EP 3 443 491 B1**

(12) **FASCICULE DE BREVET EUROPEEN**

(45) Date de publication et mention de la délivrance du brevet:
**31.07.2024 Bulletin 2024/31**

(21) Numéro de dépôt: **17716254.2**

(22) Date de dépôt: **12.04.2017**

(51) Classification Internationale des Brevets (IPC):
*A61B 5/00* (2006.01)    *A61B 5/316* (2021.01)
*A61B 5/349* (2021.01)    *G16C 20/30* (2019.01)
*G16H 20/10* (2018.01)    *G16H 40/63* (2018.01)
*G16H 50/20* (2018.01)    *G16H 50/30* (2018.01)
*G16H 70/40* (2018.01)

(52) Classification Coopérative des Brevets (CPC):
**A61B 5/72; A61B 5/316; A61B 5/349;
A61B 5/4848; A61B 5/7275; G16C 20/30;
G16H 20/10; G16H 40/63; G16H 50/20;
G16H 50/30; G16H 70/40;** G06F 2218/10

(86) Numéro de dépôt international:
**PCT/EP2017/058714**

(87) Numéro de publication internationale:
**WO 2017/178503 (19.10.2017 Gazette 2017/42)**

(54) **MÉTHODE DE DÉTERMINATION DE LA SUSCEPTIBILITÉ D'INDUCTION D'UNE TORSADE DE POINTES**

VERFAHREN ZUR BESTIMMUNG DER WAHRSCHEINLICHKEIT DER INDUZIERUNG VON TORSADES DE POINTES

METHOD FOR DETERMINING THE LIKELIHOOD OF TORSADES DE POINTES BEING INDUCED

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **13.04.2016 FR 1653246**

(43) Date de publication de la demande:
**20.02.2019 Bulletin 2019/08**

(73) Titulaires:
- **Assistance Publique - Hôpitaux de Paris**
  **75012 Paris (FR)**
- **Institut National de la Santé et de la Recherche Médicale (INSERM)**
  **75013 Paris (FR)**
- **Sorbonne Université**
  **75006 Paris (FR)**

(72) Inventeurs:
- **SALEM, Joe-Elie**
  **75015 Paris (FR)**
- **TREGOUET, David-Alexandre**
  **94350 Villiers Sur Marne (FR)**

- **HULOT, Jean-Sébastien**
  **92410 Ville D'Avray (FR)**
- **FUNCK-BRENTANO, Christian**
  **75014 Paris (FR)**
- **GERMAIN, Marine**
  **93100 Montreuil (FR)**

(74) Mandataire: **Flesselles, Bruno F.G.**
  **BF IP**
  **36 rue Jean de la Fontaine**
  **75016 Paris (FR)**

(56) Documents cités:
**US-A1- 2008 188 761**

- **COUDERC J-P. ET AL: "T-wave morphology abnormalities in benign, potent, and arrhythmogenic linhibition", HEART RHYTHM, ELSEVIER, US, vol. 8, no. 7, 1 February 2011 (2011-02-01), pages 1036 - 1043, XP028235697, ISSN: 1547-5271, [retrieved on 20110209], DOI: 10.1016/J.HRTHM.2011.02.005**

- RECANATINI M. ET AL: "QT prolongation through hERG K+ channel blockade: Current knowledge and strategies for the early prediction during drug development", MEDICINAL RESEARCH REVIEWS, vol. 25, no. 2, 1 March 2005 (2005-03-01), US, pages 133 - 166, XP055327504, ISSN: 0198-6325, DOI: 10.1002/med.20019
- CLARK M. ET AL: "Fragment-Based Prediction of the Clinical Occurrence of Long QT Syndrome and Torsade de Pointes", JOURNAL OF CHEMICAL INFORMATION AND MODELING, vol. 49, no. 11, 23 November 2009 (2009-11-23), US, pages 2617 - 2626, XP055327490, ISSN: 1549-9596, DOI: 10.1021/ci900116q
- BOTTINO D. ET AL: "Preclinical cardiac safety assessment of pharmaceutical compounds using an integrated systems-based computer model of the heart", PROGRESS IN BIOPHYSICS AND MOLECULAR BIOLOGY, vol. 90, no. 1-3, 1 January 2006 (2006-01-01), AMSTERDAM, NL, pages 414 - 443, XP055327526, ISSN: 0079-6107, DOI: 10.1016/j.pbiomolbio.2005.06.006
- SUZUKI S. ET AL: "In silico risk assessment for drug-induction of cardiac arrhythmia", PROGRESS IN BIOPHYSICS AND MOLECULAR BIOLOGY, ELSEVIER, AMSTERDAM, NL, vol. 98, no. 1, 1 September 2008 (2008-09-01), pages 52 - 60, XP024339920, ISSN: 0079-6107, [retrieved on 20080529], DOI: 10.1016/J.PBIOMOLBIO.2008.05.003

**Description**

**DOMAINE TECHNIQUE**

La présente invention se rapporte au domaine de la cardiologie, et plus particulièrement impliquant une nouvelle formule utilisable notamment dans un procédé permettant de déterminer si un médicament est susceptible d'induire des troubles de la repolarisation ventriculaire cardiaque, associés ou non associés à un trouble du rythme ventriculaire de type Torsades de Pointes.

**ETAT DE LA TECHNIQUE ANTERIEUR**

On appelle torsades de pointes un type particulier de tachycardie ventriculaire c'est à dire un trouble du rythme des ventricules cardiaques se traduisant par des accélérations du rythme cardiaque à type de tachycardie (différent néanmoins de la fibrillation ventriculaire).

Il existe deux types de torsades de pointes : les torsades à QT long et les tachycardies ventriculaires polymorphes à QT intercritique normal avec aspect de torsade.

Les torsades de pointes induites par les médicaments sont un problème de santé publique à l'échelle individuelle et à l'échelle sociétale. Dans la grande majorité des cas, le mécanisme d'action de cet effet indésirable est l'inhibition d'un canal potassique impliqué dans la repolarisation ventriculaire appelé IKr qui induit une prolongation de la phase de repolarisation et se traduit électrocardiographiquement notamment par un allongement de l'intervalle QT corrigé sur la fréquence cardiaque (QTc).

Ainsi que mentionné, dans Sekarski et al (Paediatrica, Vol. 19 No. 4 2008), l'allongement de l'intervalle QT peut provoquer une arythmie ventriculaire pouvant dégénérer et aboutir à une mort subite. L'allongement du QT constitue ainsi, actuellement, une des causes les plus fréquentes de restriction d'usage et de retrait de médicaments du marché. Parmi les médicaments ainsi retirés du marché, cette publication cite notamment le cisapride, la terfénadine, le dropéridol ou le sertindole.

Ces phénomènes sont particulièrement observés avec les antiarythmiques de classe III, qui bloquent en priorité les canaux potassiques, allongeant ainsi la repolarisation. Les antiarythmiques de classe III actuellement sur le marché possèdent tous une structure de base incluant un groupement méthanesulfoaniline (ou un bio-isostère de celui-ci). On peut ainsi citer l'amiodarone (Cordarone®), l'azimilide, le brétylium, le clofilium, le dofétilide, l'ibutilide (Corvert®), le sématilide le sotalol (Sotalex®) et le dronédarone (Multaq®).

La publication ci-dessus présente, dans les tableaux 1 et 2, un certain nombre de médicaments connus pour allonger le QT. Cette publication rappelle également que le « *University of Arizona Center for Education and Research on Therapeutics* » a établi et maintient une base de données de ces médicaments que l'on peut retrouver sur leur site internet www.torsades.org ou https://crediblemeds.org/.

Dans le processus de dépôt de dossier pour autorisation de mise sur le marché d'un nouveau médicament, toutes les molécules doivent avoir une évaluation de leur potentiel torsadogénique (potentiel d'induire une torsade de pointes). Cette évaluation passe, chez l'homme par des études approfondies des modifications induites par les nouveaux médicaments de la repolarisation ventriculaire et particulièrement de l'allongement de l'intervalle QT, ou de l'allongement corrigé QTc. Cet allongement de l'intervalle QTc est le marqueur actuellement recommandé pour estimer le degré d'inhibition d'lKr d'un médicament administré à des sujets avec une repolarisation ventriculaire normale à l'état basale.

Ainsi, on considère qu'une molécule test est à risque significatif justifiant une surveillance rapprochée de ce risque rythmique lors de la mise sur le marché lorsque l'augmentation de QTc est supérieure à 20 ms. Lorsque l'allongement de QT/QTc est compris entre 5 et 20 ms, on se trouve dans une « zone grise » et des études complémentaires doivent être réalisées pour mener à une conclusion plus fiable. On considère généralement qu'une molécule est sûre pour ce qui est de la repolarisation cardiaque lorsque l'on observe un allongement de QT/QTc inférieur à 5ms.

Toutefois, l'allongement de cet intervalle QTc est un paramètre très imparfait, peu spécifique de l'inhibition du canal IKr ventriculaire. Ainsi, si une inhibition du canal IKr augmente l'intervalle QT, celui-ci peut être augmenté sans inhibition d'iKr, le risque de torsade de pointes d'origine médicamenteuse étant alors moins important. Certaines molécules utiles vont ainsi voir leur développement interrompu à cause d'une surestimation de ce risque quand d'autres molécules vont voir leur développement poursuivi malgré un risque torsadogénique sous-estimé.

À l'échelle individuelle, les torsades de pointes médicamenteuses peuvent affecter des sujets sans grande comorbidité et conduire au décès. Les déterminants de ce risque individuel sont encore mal connus avec notamment un sur-risque identifié en cas d'hypokaliémie, de QT long congénital mais il n'existe pas de test diagnostic fiable permettant d'évaluer ce risque personnel surtout en cas de normalité de l'intervalle QTc à l'état de base.

Ainsi, tant les instances réglementaires (comme la Food and Drug Administration) que les médecins cliniciens sont à la recherche de nouvelles méthodes plus pertinentes et prédictives du risque torsadogénique des médicaments, dans l'évaluation pré-marketing, ou pour un patient particulier, qui aille au-delà de la simple analyse de l'augmentation de la durée de l'intervalle QTc après stress pharmacologique.

Le sotalol est un médicament antiarythmique recommandé et utilisé pour contrôler le rythme chez des patients avec fibrillation atriale. Le sotalol a une capacité élevée à bloquer le canal potassique IKr, associé à un risque dose dépendant d'induction de torsades de pointes, atteignant jusqu'à 7% des patients traités. Le sotalol a une pharmacocinétique linéaire, une absorption pratiquement complète sans grande variabilité, et une concentration maximale observée 3 à 5 heures après prise orale.

Couderc et al (Heart Rhythm. 2011 Jul;8(7):1036-43) décrivent l'analyse de tracés et de valeurs QTc, Tamp ou TpTe à divers moments, mais ne s'intéresse pas à la dynamique des paramètres après traitement ; ainsi, ce document ne calcule pas les valeurs ΔQTc, ΔTamp et ΔTpTe telles qu'envisagées dans la présente demande, ni le calcul d'une composante principale sur la base de ces valeurs.

US 2008/188761 décrit une méthode pour détecter des retards du canal potassique par comparaison de la corrélation entre les Tamp et le rythme cardiaque, mais ne décrit pas plus les valeurs ΔQTc, ΔTamp et ΔTpTe telles qu'envisagées dans la présente demande, ni le calcul d'une composante principale sur la base de ces valeurs.

Recanatini et al (Med Res Rev. 2005 Mar;25(2):133-66) décrivent, dans un article de revue, le lien entre l'allongement du QT et les torsades de pointes, mais ne mentionnent, ni ne suggèrent la stratégie envisagée dans la présente demande.

Clark et al (J Chem Inf Model. 2009 Nov;49(11):2617-26) décrivent une méthode pour déterminer l'association entre le QT long et les torsades de pointes pour diverses molécules, mais ne mentionnent, ni ne suggèrent la stratégie envisagée dans la présente demande.

Bottino et al (Prog Biophys Mol Biol. 2006 Jan-Apr;90(1-3):414-43) décrivent une méthode utilisable pour analyser si certains médicaments sont susceptibles d'induire des torsades de pointes, mais ne mentionnent, ni ne suggèrent la stratégie envisagée dans la présente demande.

Suzuki et al (Prog Biophys Mol Biol. 2008 Sep;98(1):52-60) décrivent des méthods *in silico* pour déterminer si une molécule est susceptible d'induire une arythmie cardiaque, mais ne mentionnent, ni ne suggèrent la stratégie envisagée dans la présente demande.

Il existe donc un besoin de mettre en place un test qui permette de prédire le risque qu'un médicament puisse induire une torsade de pointes, après administration chez des patients.

Alternativement, il est également intéressant de déterminer si un patient particulier possède un risque spécifique de présenter une torsade de pointes, après administration d'un médicament spécifique.

**EXPOSE DE L'INVENTION**

L'invention se propose de fournir une méthode mise en oeuvre par ordinateur telle que revendiquée permettant de déterminer aisément, après une simple mesure d'électrocardiogramme, si un patient est susceptible de présenter un événement de torsade de pointes après administration d'un médicament.

Les inventeurs ont ainsi mis en évidence le fait qu'il est possible d'obtenir un résultat quantitatif fiable de la capacité, pour une substance d'inhiber le canal potassique IKr (effet général de la substance) ou pour un patient de présenter une telle inhibition du canal potassique IKr après administration d'une substance susceptible de l'inhiber (effet de réponse individuelle du patient), en étudiant, non seulement le QTc, mais deux valeurs supplémentaires, l'amplitude maximale de l'onde T (TAmp) et la durée entre le pic et la fin de l'onde T (TpTe).

En combinant les variations observées entre les trois valeurs mentionnées ci-dessus, les inventeurs ont défini un algorithme permettant de fournir une information précise quant au risque de survenue d'un trouble de la repolarisation cardiaque induite par une substance.

L'algorithme proposé par les inventeurs est particulièrement intéressant, en ce qu'il permet à la fois d'obtenir des informations sur une substance ou composition particulière, lorsque testée sur une cohorte de patients, mais également de déterminer le risque individuel pour un patient particulier, ce qui permet d'effectuer un suivi adéquat des patients à risque, en diminuant le coût de suivi des patients non à risque *a priori*.

**Mesures utilisées dans le contexte de l'invention**

On utilise ainsi les mesures suivantes :

TAmp: amplitude maximale du premier pic de l'onde T

On calcule la différence (exprimée en pourcentage) entre l'amplitude maximale du premier pic de l'onde T avant et après administration de la substance que l'on souhaite tester. De fait, en cas de « notch », on peut observer deux pics de l'onde T. Il convient de mesurer l'amplitude du premier pic, même si elle est inférieure à celle du deuxième.

La donnée calculée est donc ΔTamp(%)=((Tamp avant administration - TAmp après administration)/(Tamp avant administration))*100

On calcule ici, la différence entre l'amplitude de l'onde T avant administration et celle après administration afin que ΔTAmp soit positif. Cela est dû au fait que l'inhibition du canal IKr induit une diminution de l'amplitude de l'onde T. Le fait d'effectuer le calcul ci-dessus permet de refléter cette diminution en pourcentage. L'expression des résultats en valeurs absolues (mV) permet également de calculer un algorithme opératoire.

On aurait toutefois pu calculer la différence entre l'amplitude de l'onde T après administration et celle mesurée avant administration et ΔTAmp aurait été négatif.

La valeur de TAmp que l'on prend en considération dans cette formule est préférentiellement une moyenne de plusieurs mesures de TAmp.

Les mesures peuvent être réalisées sur les dérivations que choisira un praticien, mais préférentiellement sur les dérivations D2, V2, V3, On peut également effectuer ces mesures sur les dérivations V4 ou V5, en particulier, si TAmp avant administration de la substance est inférieur à 0.1 mV.

Dans un mode de réalisation particulier, et pour diminuer les erreurs potentielles dues aux variations pouvant exister chez le patient au cours de la mesure d'ECG, on préfère mesurer la valeur de TAmp sur un complexe moyenné (généralement généré par un complexe médian type (template) obtenu à partir des enregistrements réalisés pendant 10 secondes), voire sur la moyenne de trois complexes moyennés générés à quelques minutes d'intervalle.

TpTe : Intervalle entre le premier pic maximal de l'onde T et la fin de l'onde T (TpTe)

On calcule la différence (exprimée en pourcentage) entre le premier pic maximal de l'onde T et la fin de l'onde T, avant et après administration de la substance que l'on souhaite tester.

La donnée calculée est donc ΔTpTe(%)=((TpTe après administration - TpTe avant administration)/(TpTe avant administration))*100

La valeur de TpTe que l'on prend en considération dans cette formule est préférentiellement une moyenne de plusieurs mesures de TpTe.

Les mesures peuvent être réalisées sur les dérivations que choisira un praticien, mais préférentiellement sur les dérivations V2, V3, V4. On peut également effectuer les mesures sur les dérivations V5 ou V6.

Dans un mode de réalisation particulier, et pour diminuer les erreurs potentielles dues aux variations pouvant exister chez le patient au cours de la mesure d'ECG, on préfère mesurer la valeur de TpTe sur un complexe moyenné (généralement généré par un complexe médian type (template) obtenu à partir des enregistrements réalisés pendant 10 secondes), voire sur la moyenne de trois complexes moyennés générés à quelques minutes d'intervalle.

On peut également utiliser la valeur absolue (en msec) de la variation TpTe dans le cadre de l'algorithme.

QTcF : intervalle QT corrigé sur la fréquence cardiaque (OTc)

On calcule la différence (exprimée en pourcentage) entre l'intervalle QT corrigé sur la fréquence cardiaque par une méthode appropriée qui minimise l'influence de la fréquence cardiaque sur la valeur de QTc, c'est-à-dire établit une pente non différente de zéro dans la relation QTc en fonction de la fréquence cardiaque (le plus souvent, chez l'adulte, la correction appropriée se fait par la méthode de Fridericia, QTcF), avant et après administration de la substance que l'on souhaite tester.

On rappelle que

$$QTcF = \frac{QT}{\sqrt[3]{RR}}$$

RR étant l'intervalle entre le début d'un complexe QRS et le début du complexe QRS suivant exprimé en seconde.

La donnée calculée, pour l'utilisation du QTcF, est donc ΔQTcF(%)=((QTcF après administration - QTcF avant administration) / (QTcF avant administration))*100

La valeur de QTcF que l'on prend en considération dans cette formule est préférentiellement une moyenne de plusieurs mesures de QTcF, en particulier une moyenne d'au moins trois complexes consécutifs. Ainsi que pour les autres valeurs vues plus haut, on peut aussi utiliser la valeur QTcF que l'on obtient sur un complexe médian obtenu à partir d'une enregistrement de quelques secondes, ou une moyenne de complexes moyennés.

Les mesures peuvent être réalisées sur les dérivations que choisira un praticien, mais préférentiellement sur la dérivation D2.

On pourrait toutefois également calculer QTc par toute autre méthode connue dans l'art, en particulier la méthode de Bazett (QTcB) :

$$QTcB = \frac{QT}{\sqrt{RR}}$$

Là encore, on peut utiliser la valeur absolue de $\Delta$QTc, plutôt que la variation en pourcentage.

D'une façon générale, on préfère calculer les $\Delta$TAmp, $\Delta$QTc, $\Delta$TpTe de telle sorte qu'ils aient tous le même signe.

Algorithme à mettre en oeuvre dans le contexte de l'invention

L'algorithme (formule) proposé ci-dessous permet d'obtenir une valeur quantitative, nommée PC1, très bien associée à l'apparition de signe ECG déterminant une inhibition d'IKr, et présumés associés à un risque accru d'épisode de torsade de pointes à QT long.

PC1= a1 x $\Delta$TAmp(%) + a2 x $\Delta$QTcf(%) + a3 x $\Delta$TpTe(%) + b

dans lequel

- $-0,05 \leq a1 \leq -0,030$

- $-0,2 \leq a2 \leq -0,1$

- $-0,04 \leq a3 \leq -0,02$

- $1,5 \leq b \leq 2$

De préférence,

- $-0,040 \leq a1 \leq -0,035$

- $-0,16 \leq a2 \leq -0,145$

- $-0,35 \leq a3 \leq -0,025$

- $1,7 \leq b \leq 1,9$

Dans un mode de réalisation préféré,

$$a1 = -0,038 \pm 0,002$$

$$a2 = -0,15 \pm 0,001$$

$$a3 = -0,031 \pm 0,002$$

$$b = 1,79 \pm 0,05$$

Dans un mode de réalisation préféré,

$$a1 = -0,03814$$

$$a2 = -0,15074$$

$$a3 = -0,03059$$

$$b = 1,79249$$

Dans un mode de réalisation particulier,

$$PC1= -0,038 \times \Delta TAmp(\%) -0,151 \times \Delta QTcf(\%) -0,0306 \times \Delta TpTe(\%) + 1,792$$

Plus la valeur PC1 est négative, plus la substance administrée, inhibe le canal IKr et plus le patient est présumé présenter un risque de faire un épisode de torsade de pointes.

Ces algorithmes et valeurs sont valables lorsque l'on calcule ΔTAmp ainsi qu'indiqué ci-dessus pour qu'il soit positif. Si on calcule ΔTAmp de façon à ce qu'il soit négatif, les valeurs de l'algorithme changeront, et un nouvel algorithme pourra être défini en utilisant la méthode décrite plus bas. De même, si l'on utilise les valeurs absolues des différences entre TAmp, TpTe et QTc, on obtiendra un algorithme présentant d'autres valeurs.

Évaluation du potentiel torsadogénique d'un médicament

Ainsi que vu plus haut, il est important de pouvoir évaluer le potentiel torsadogénique d'un composé, lors des essais cliniques préalables à l'obtention d'une autorisation de mise sur le marché, afin de pouvoir rédiger les éventuelles précautions d'utilisation de ce médicament.

Le potentiel torsadogénique d'une substance est le potentiel que cette substance induise une torsade de pointes après administration chez un patient.

La FDA (*Food and Drug Administration*) et l'EMA (*European Medicine Agency*) ont publié des directives issues de la conférence internationale pour l'harmonisation (ICH) sur la façon d'effectuer ces essais sous le titre *E14 Clinical Evaluation* of QT/QTc *Interval Prolongation and Proarrhythmic Potential for Non-Antiarrhythmic Drugs.*

Ces directives sont accessibles sur le site de cette agence, et accompagnées de questions et réponses.

L'adresse des directives est : http://www.fda.gov/downloads/drugs/guidancecomplianceregulatoryinformation/gui dances/ucm073153.pdf

La capacité d'allonger la repolarisation cardiaque par des médicaments ou substances non antiarythmiques peut être mesurée par l'allongement de l'intervalle QT sur l'électrocardiogramme (ECG) de surface. Il est rappelé que cet intervalle QT, représente la durée de la dépolarisation et la repolarisation subséquente ventriculaire et est mesuré à partir du début du complexe QRS à la fin de l'onde T. Un retard de repolarisation cardiaque crée un environnement favorisant le développement des arythmies cardiaques, la plupart clairement une torsade de pointes (TdP), mais éventuellement d'autres tachyarythmies ventriculaires aussi.

Ainsi que vu plus haut, l'intervalle QT est un biomarqueur imparfait pour le risque d'effet proarythmique, même s'il existe, dans la population générale, une relation qualitative entre l'intervalle QT et le risque de torsade de pointes, surtout pour les médicaments qui provoquent un allongement substantiel de l'intervalle QT.

D'une façon générale, on corrige la valeur de l'intervalle QT mesuré (obtention du QTc) pour tenir compte de la fréquence cardiaque, cet intervalle QT présentant une relation inverse avec celle-là. Il n'est toutefois pas clair si le développement de l'arythmie est plus étroitement lié à une augmentation de l'intervalle QT, de façon absolue, ou du QTc.

La plupart des médicaments présentant un risque d'induction de torsade de pointes augmentent l'intervalle QT absolu et le QTc.

De façon générale, lorsque la molécule test augmente le QTc d'une durée inférieure à 5 ms, on considère que celle-ci est sûre sur cet aspect. Lorsqu'elle augmente le QTc d'une durée supérieure à 20 ms, les effets de la molécule devront faire l'objet d'une surveillance post-marketing. Lorsque la molécule augmente le QTc d'une durée comprise entre 5 ms et 20 ms, on se trouve dans une « zone grise », c'est-à-dire qu'il n'est pas possible de conclure *a priori* quant au risque réel de torsade de pointes, et que des analyses et études complémentaires sont requises.

Il est donc requis de caractériser rigoureusement les effets de nouveaux médicaments quant au risque de retarder la repolarisation cardiaque. L'algorithme défini ci-dessus permet, en particulier si les études sont réalisées avec le Sotalol à 80mg en tant que témoin positif, de donner des informations complémentaires intéressantes dans la zone grise d'augmentation du QT/QTc comprise entre 5 et 20 ms.

À ce stade, l'étude est réalisée sur une cohorte de patients. Effectuer ces études sur un nombre de patients suffisant permet ainsi d'obtenir des résultats qui présentent une réalité statistique pour la molécule que l'on souhaite tester (médicament d'intérêt), éliminant les variations inter-patients.

On comparera préférentiellement un placébo et la substance d'intérêt afin de déterminer si cette substance allonge l'intervalle QT (on pourrait mesurer les intervalles sans utiliser de placébo, mais il est préférable d'en utiliser un, afin de tenir compte de l'évolution naturelle du QT au cours de la journée).

Avantageusement, on utilisera également des contrôles positifs pour l'allongement de l'intervalle QT. Ainsi, dans les directives de la FDA, celle-ci conseille d'utiliser un contrôle positif qui augmente le QT/QTc d'environ 5ms, afin de vérifier que cet effet de faible amplitude est bien détecté. On peut utiliser deux contrôles positifs, l'un augmentant l'intervalle QT en induisant une inhibition manifeste du canal potassique IKr (tel le sotalol), et un autre contrôle augmentant l'intervalle QT sans inhibition importante du canal IKr, tel que la moxifloxacine orale. On peut utiliser, en tant que contrôle, tout médicament connu pour allonger le QT, notamment tel que cité dans les tableaux 1 ou 2 de Sekarski *et al.*

Toutefois, du fait des risques potentiels posés par ces médicaments des tableaux 1 et 2 de Sekarski *et al,* on utilise de préférence le sotalol à 80 mg en tant que témoin positif. Cette molécule, à cette concentration, présente de nombreux avantages :

- la concentration maximale est bien connue (environ trois heures après administration orale), ce qui permet de planifier aisément le moment auquel faire les mesures mentionnées ci-dessus.
- Cette molécule est connue pour inhiber le canal IKr
- Cette molécule, à cette concentration augmente le QT/QTc d'environ 20 ms.
- Cette molécule, à cette concentration, n'induit pas d'épisode de torsade de pointes, et est donc sûre pour le patient. Il est toutefois connu que cette molécule, à ses doses utilisées en clinique (utilisée jusqu'à 320 mg), peut induire des torsades de pointes.

L'étude est réalisée sur une cohorte de patients, selon un protocole qui peut être le suivant, pour chaque patient :

- On effectue les mesures des TAmp, TpTe, QTc sur le patient avant d'administrer la substance dont on souhaite étudier le potentiel torsadogénique
- On effectue les mesures des mêmes paramètres TAmp, TpTe, QTc sur le patient, après administration de la substance (qui peut donc être le placébo, la substance d'intérêt, ou le contrôle positif).

On peut effectuer une seule mesure de ces paramètres (si l'on connaît, pour la substance, la pharmacocinétique, et le pic de concentration de la substance dans la circulation du patient), mais on effectue, de préférence plusieurs mesures à plusieurs intervalles de temps (par exemple tous les ¼ heures ou toutes les heures, en fonction de la demi-vie de la molécule).

Le fait de relever périodiquement les TAmp, TpTe et QTc permet d'améliorer la possibilité de détecter l'effet torsadogénique potentiel de la substance, voire de déterminer à quel moment cet effet est le plus important.

- On calcule les valeurs ΔTAmp, ΔTpTe, ΔQTc, selon les formules indiquées ci-dessus.
- On utilise l'algorithme mentionné ci-dessus, afin de calculer le résultat pour chaque substance testée.

D'une façon générale, l'homme du métier sera capable de définir le protocole adéquat en fonction de la substance d'intérêt (moments auxquels on mesurera les TAmp, TpTe et QTc), en tenant compte notamment des directives de la FDA telles que mentionnées ci-dessus. En particulier, si l'on utilise le sotalol en tant que contrôle positif (d'inhibition du canal IKr), on mesure les valeurs ci-dessus trois heures après administration, ce qui correspond au pic le plus fréquent de concentration de cette substance. Si l'on utilise une autre substance en tant que contrôle, on choisit, pour mesurer les valeurs ci-dessus, le moment le plus adéquat, correspondant à celui où l'effet sur la dépolarisation est le plus important. Ces contrôles étant connus, l'homme du métier sait donc déterminer ces moments. De même, les concentrations optimales des contrôles utilisés seront déterminées par l'homme du métier en fonction des connaissances qu'il aura sur les molécules contrôles qu'il utilisera. Ainsi, il pourra utiliser le sotalol à la dose à 80 mg pour les raisons de risque rythmique exposées ci-dessus. L'utilisation d'une quantité supérieure de sotalol pourrait éventuellement être envisagée, mais n'est toutefois pas préférée du fait des risques pour les patients et de sa non-conformité aux recommandations d'initiation du traitement chez les malades qui en ont une indication.

Dans la définition du protocole par l'homme du métier, on préfère effectuer les différentes mesures au même moment du cycle menstruel, lorsque que la patiente est de sexe féminin. En effet, on sait que le niveau d'hormones influence le risque, chez une femme, de troubles de la repolarisation (QT long).

D'une façon générale, ainsi qu'indiqué ci-dessus, on effectue les étapes ci-dessus pour chaque patient d'une cohorte de patients, et l'on obtient ainsi une valeur calculée par l'algorithme décrit ci-dessus pour chaque patient (et pour chaque moment auquel les paramètres TAmp, TpTe, QTc ont été mesurés), et pour chaque substance.

Pour chaque moment auquel les paramètres TAmp, TpTe, QTc ont été mesurés, on préfère effectuer une moyenne des valeurs obtenues pour chaque patient, et pour chaque substance administrée (substance d'intérêt et éventuellement les placébo, et contrôle(s) positif(s)). Ceci permet de s'affranchir de « l'effet patient », c'est-à-dire le fait que deux patients sont susceptibles de réagir différemment à un médicament ou une substance donnée.

La valeur de la moyenne des valeurs PC1 est alors une valeur qui prend plus en compte l'effet torsadogénique présumé réel de la substance d'intérêt.

Il est préférable que la cohorte de patients (le nombre de patients sur lesquels on teste la substance d'intérêt) soit au minimum de 10 patients, de préférence au minimum de 20 voire de 50 ou de 100 patients. L'homme du métier sera capable de déterminer le nombre minimal de sujets dans la cohorte de patients afin d'obtenir des résultats qui soient statistiquement significatifs.

De même, avant de réaliser ces études, il est préférable que l'homme du métier ait réalisé des études de pharmacocinétiques et de réponse en fonction des doses de la substance d'intérêt, afin de déterminer les meilleurs moments pour mesurer les trois valeurs physiologiques mentionnées ci-dessus.

Afin de déterminer le potentiel torsadogénique de la substance d'intérêt, l'analyse sera différente, selon que l'on a utilisé ou non des contrôles.

*Si l'on a utilisé le sotalol en tant que contrôle positif d'inhibition du canal IKr*

La valeur PC1 est calculée pour chaque patient, pour le placébo, le sotalol trois heures après administration, et la substance d'intérêt (molécule test), aux moments déterminés par le praticien, en fonction des caractéristiques pharmacocinétiques de la molécule test.

On effectue ensuite une moyenne de ces valeurs PC1 pour chacune des molécules (test, Sotalol, placébo), ainsi que la médiane.

Si la moyenne, ou alternativement la médiane de PC1 pour le groupe de la molécule test est significativement inférieure à celle obtenue pour le groupe sotalol, cette molécule test induit donc une inhibition du canal IKr au-delà d'un risque jugé raisonnable et devra faire l'objet de tests complémentaires et d'une surveillance accrue après mise sur le marché.

Si la moyenne de PC1 pour le groupe de la molécule test est non significativement différente à celle obtenue pour le placébo alors la molécule peut être considérée comme n'étant pas à risque.

Si la moyenne de PC1 pour le groupe de la molécule test est significativement inférieure à celle calculée pour le placébo, mais est non significativement différente de la moyenne de PC1 pour le sotalol, on se trouve alors dans une « zone grise ». Un risque torsadogène existe puisqu'il existe avec le sotalol, potentiellement, du moins, à des doses plus élevées.

On devra alors mettre en place les surveillances d'usage si l'on a observé une augmentation de QT/QTc supérieure à 5 ms pour la molécule test par rapport au placébo.

L'analyse est équivalente si l'on a utilisé un autre contrôle positif que le sotalol, ce contrôle devant toutefois induire une augmentation de QT et une inhibition du canal IKr avec un risque torsadogénique considéré plus ou moins équivalent à celui associé à 80mg de Sotalol.

Si *l'on n'a* pas *utilisé de contrôle positif*

On peut également utiliser l'algorithme défini ci-dessus en l'absence de contrôle positif, même s'il n'est pas conseillé de ne pas utiliser de contrôle positif.

Dans ce cas, il est préférable que la population dans laquelle les essais sont réalisés contienne environ 50% de femmes et 50% d'hommes, bien que l'on puisse aller jusqu'à 60 % de femmes et 40 % d'hommes (ou 40 % de femmes et 60 % d'hommes), selon les directives de la FDA. Les patients sont généralement âgés de 18 à 60 ans.

Du fait du risque supérieur, pour les femmes, de développer des troubles de la repolarisation, par rapport aux hommes, l'utilisation d'une cohorte de patients présentant la répartition ci-dessus permet d'augmenter la pertinence du résultat de l'algorithme et la confiance que l'on peut tirer des résultats obtenus, et l'analyse que l'on peut en faire.

La valeur PC1 est calculée pour chaque patient, pour le placébo, et la substance d'intérêt (molécule test), aux moments déterminés par le praticien, en fonction des caractéristiques pharmacocinétiques de la molécule test.

Si la moyenne de PC1 pour le groupe de la molécule test est non significativement différente de celle obtenue pour le placébo alors la molécule peut être considérée comme n'étant pas à risque.

Si la moyenne de PC1 pour le groupe de la molécule test est significativement supérieure de celle obtenue pour le placébo alors la molécule peut être considérée comme n'étant pas à risque.

Si la moyenne de PC1 pour le groupe de la molécule test est négative, et significativement inférieure à celle obtenue pour le groupe placébo, cette molécule test est à risque et devra faire l'objet de tests complémentaires et d'une surveillance accrue après mise sur le marché.

Si la moyenne de PC1 pour le groupe de la molécule test est négative, et non significativement inférieure à celle calculée pour le placébo, on se trouve alors dans une « zone grise ». On devra alors mettre en place les surveillances d'usage si l'on a observé une augmentation de QT/QTc supérieure à 5 ms pour la molécule test par rapport au placébo.

En cas de potentiel torsadogénique déterminé par la méthode décrite ci-dessus ou si la molécule est dans la zone grise, on effectuera des études plus poussées pour confirmer ce potentiel et mieux le qualifier et le quantifier.

En l'absence de contrôle positif, il est préférable d'effectuer les études sur un nombre de patients suffisants pour effectuer les analyses statistiques appropriées.

Évaluation du risque individuel d'un patient de présenter un événement de torsade de pointes

L'algorithme décrit ci-dessus permet également d'évaluer le risque individuel d'un patient à présenter un événement de torsade de pointes, après administration d'une substance ayant un potentiel torsadogénique.

Pour ce faire, on peut suivre le protocole suivant :

- On effectue les mesures des TAmp, TpTe, QTcF sur le patient avant d'administrer du sotalol
- On effectue les mesures des mêmes paramètres TAmp, TpTe, QTcF sur le patient, trois heures après administration de sotalol. Ainsi que vu plus haut, on sait que le sotalol présente une concentration maximale environ 3 heures après prise orale, et on préfère utiliser une dose unique de 80 mg.

- On calcule les valeurs ΔTAmp, ΔTpTe, ΔQTcF, selon les formules indiquées ci-dessus.
- On applique l'algorithme ci-dessus à ces valeurs, afin d'obtenir un résultat PC1 final.

L'analyse des résultats s'effectue de la façon suivante :

- Si PC1 ≤ -3.2 : un très haut risque de présenter un événement de torsade de pointes (cette valeur correspond au 5ème percentile chez les femmes qui sont plus à risque que les hommes, c'est-à-dire que seules 5% de femmes ont une PC1 inférieure cette valeur)
- Si -3.2 < PC1 ≤ -2.4 : le patient a un risque élevé, mais un peu moins important (compris entre les 5ème et 10ème percentile des femmes)
- Si -2.4 < PC1 ≤ -1.25 : le patient présente un risque moyen (correspondant au décile-quartile chez les femmes)
- Si -1.25 < PC1 ≤ -0.48 : le patient présent un risque faible, correspondant au quartile-45ème percentile pour les femmes et au 5ème percentile pour les hommes
- Si -0.48 < PC1 ≤ -0.22 : le risque du patient est très faible, cet intervalle correspondant au 45ème-53ème percentile pour les femmes et au 5ème-10ème percentile pour les hommes
- Si PC1 > -0,22, on peut considérer que le patient ne présente pas de risque

De préférence, on utilise une quantité faible de sotalol, c'est-à-dire équivalente à la quantité généralement administrée à l'initiation du traitement en clinique. Ainsi, il est préféré que la dose de sotalol administrée au patient soit de 80 mg (prise unique par voie orale), ce qui permet d'observer un trouble de la repolarisation sans mettre en danger le patient. En effet, on sait que le sotalol peut inhiber le canal potassique IKr. Les algorithmes décrits ci-dessus ont été validés sur des cohortes de patients ayant reçu une dose unique de 80mg de sotalol, les mesures ayant été faites trois heures après administration. Les valeurs obtenues sont des valeurs quantitatives fiables corrélées à l'inhibition du canal potassique IKr.

Ainsi, le résultat de l'algorithme permet d'indiquer si le patient est, ou non, susceptible de réagir à un médicament ayant la capacité d'inhiber le canal IKr. Ce test est donc plus informatif que la simple mesure du QT/QTc.

En fonction du résultat obtenu et du risque ainsi déterminé pour le patient, l'homme du métier sera capable d'ajuster le suivi nécessaire du patient.

Ceci permet donc de disposer d'un nouvel outil permettant de générer une variable quantitative de l'inhibition du canal potassique IKr.

Il peut être utilisé une méthode ex *vivo* ou *in vitro* pour déterminer le risque d'induction d'un effet torsadogénique après administration d'une composition chez un patient comprenant les étapes :

I. combiner, dans un algorithme, obtenu par la méthode suivante :

    a. rassembler une cohorte contenant un nombre n de patients, n étant supérieur ou égal à 50, 75, voire 100 ;
    b. leur administrer un médicament inhibant le canal IKr;
    c. mesurer les QTc, Tamp, TpTe avant et après administration de ce médicament avec

        i. QTc = intervalle QT corrigé sur la fréquence cardiaque
        ii. TAmp = Amplitude maximale du premier pic de l'onde T
        iii. TpTe = Intervalle entre le premier pic maximal de l'onde T et la fin de l'onde T ;

    d. calculer les ΔQTc, ΔTamp et ΔTpTe correspondant à la variation, en pourcentage, des valeurs QTc, TAmp et TpTe obtenues après administration du médicament, par rapport à celles mesurées avant administration pour chacun des patients
    e. effectuer une analyse en composantes principales sur les valeurs ΔQTc, ΔTamp et ΔTpTe afin de générer trois nouvelles variables quantitatives PC1, PC2 et PC3
    f. ledit algorithme étant la formule permettant d'obtenir la variable PC1 à partir des ΔQTc, ΔTamp et ΔTpTe, telle que décrite plus haut

II. Obtenir un résultat final de cette combinaison ;
III. Comparer la valeur de ce résultat final à une valeur de référence.

Dans un autre mode de réalisation, les ΔQTc, ΔTamp et ΔTpTe utilisés en I.d sont les valeurs absolues des différences, et pas les variations en pourcentage.

Dans ce mode de réalisation, la méthode est *in vitro* ou ex *vivo,* c'est-à-dire qu'elle n'est pas appliquée au corps humain. Elle utilise des données qui ont été relevées par électrocardiogramme sur un patient, mais ne comprends pas ces étapes de recueil des données. Cette méthode n'est donc pas appliquée au corps humain.

La demande décrit également une méthode pour déterminer le risque d'induction d'un effet torsadogénique après administration d'une composition chez un patient comprenant les étapes :

I. mesurer, chez le patient, les valeurs QTc (intervalle QT corrigé sur la fréquence cardiaque), TAmp (amplitude maximale du premier pic de l'onde T), TpTe (intervalle entre le premier pic maximal de l'onde T et la fin de l'onde T) avant et après administration de cette composition,
II. calculer les valeurs ΔQTc, ΔTamp et ΔTpTe correspondant respectivement à la différence, en pourcentage, de ces mesures obtenues après et avant administration
IIII. combiner, dans un algorithme, obtenu par la méthode suivante :

    a. rassembler une cohorte contenant un nombre n de patients, n étant supérieur ou égal à 50, 75, ou 100 ;
    b. leur administrer un médicament inhibant le canal IKr;
    c. mesurer les QTc, Tamp, TpTe avant et après administration de ce médicament;
    d. calculer les ΔQTc, ΔTamp et ΔTpTe correspondant à la variation, en pourcentage, des valeurs QTc, Tamp et TpTe obtenues après administration du médicament, par rapport à celles mesurées avant administration pour chacun des patients
    e. effectuer une analyse en composantes principales sur les valeurs ΔQTc, ΔTamp et ΔTpTe afin de générer trois nouvelles variables quantitatives PC1, PC2 et PC3
    f. ledit algorithme étant la formule permettant d'obtenir la variable PC1 à partir des ΔQTc, ΔTamp et ΔTpTe ,

IV. Obtenir un résultat final de cette combinaison dans l'algorithme ;

V. Comparer la valeur de ce résultat final à une valeur de référence.

Dans un autre cas de la divulgation, les $\Delta$QTc, $\Delta$Tamp et $\Delta$TpTe utilisés en II et III.d sont les valeurs absolues des différences, et pas les variations en pourcentage.

Les méthodes ainsi décrites permettent également de déterminer si la composition utilisée inhibe le canal potassique IKr chez un patient, le résultat final obtenu après application de l'algorithme étant lié quantitativement à l'inhibition du canal IKr.

L'algorithme (formule) utilisé dans les méthodes ci-dessus est celui décrit ci-dessus. L'exemple 1 décrit la façon dont il a été obtenu.

D'une façon générale, afin d'obtenir cette formule utilisable dans le cadre des méthodes décrites dans la présente demande, on suit le procédé suivant :

a. rassembler une cohorte contenant un nombre n de patients.

De préférence, le nombre n de patients suivis est supérieur ou égal à 50, 75 voire 100, afin d'obtenir des résultats qui présentent une réelle valeur statistique ; de préférence, la cohorte contient un nombre égal de femmes et hommes ou pas plus de 60 % de femmes et 40 % d'hommes ou inversement. Ceci permet d'obtenir un algorithme utilisable quel que soit le sexe du patient. Toutefois, on peut aussi réaliser cette méthode d'obtention d'un algorithme en utilisant une population de patients exclusivement féminins ou exclusivement masculins. Les algorithmes obtenus seront alors différents, du fait de l'inhibition supérieur du canal IKr observé chez les femmes par rapport aux hommes.

Dans le cadre des méthodes selon la divulgation, on utilisera alors l'un ou l'autre algorithme selon le sexe du patient sur lequel on souhaite effectuer l'étude.

b. administrer à chaque patient un médicament allongeant le QT, de préférence en inhibant le canal IKr ;

Le médicament que l'on administre à cette étape est un médicament tel que décrit ci-dessus, notamment les produites des tableaux 1 ou 2 de Sekarski et al. Ce médicament est choisi par l'homme du métier en fonction de ses caractéristiques pharmacocinétiques, et les doses sont adaptées afin notamment de ne pas mettre en danger les patients de la cohorte, tout en permettant l'observation de l'allongement du QT lié à ce médicament dans un nombre suffisant (pour analyse statistique) de patients.

On préfère ainsi utiliser le sotalol, en particulier à 80 mg par voie orale pour les raisons mentionnées plus haut.

c. mesurer les QTc, TAmp, TpTe avant et après administration de ce médicament;

Ces valeurs sont mesurées avant l'administration du médicament à l'étape b) (la mesure avant cette administration pouvant ainsi être qualifiée d'étape b0), et après cette administration. Du fait que le médicament utilisé à l'étape b est connu de l'homme du métier, la mesure après administration est effectuée à un moment optimal, déterminé par l'homme du métier en fonction des propriétés pharmacocinétiques du médicament de l'étape b), tel que l'allongement du QT induit par ce médicament est maximal. Ceci correspond en général au moment où la concentration plasmatique du médicament est maximale.

Si l'on choisit le sotalol en tant que médicament à l'étape b), on effectue les mesures 3 heures après adminis-tration.

d. calculer les $\Delta$QTc, $\Delta$Tamp et $\Delta$TpTe correspondant à la variation, en pourcentage, entre les valeurs QTc, TAmp et TpTe obtenues après administration du médicament, par rapport à celles mesurées avant administration, pour chacun des patients

Les valeurs $\Delta$QTc, $\Delta$Tamp et $\Delta$TpTe sont ainsi préférentiellement calculées selon les formules indiquées ci-dessus. On pourrait toutefois calculer $\Delta$Tamp selon la formule $\Delta$Tamp = ((TAmp après administration - Tamp avant administration)/(TAmp avant administration) $\times$ 100.

Dans un autre mode de réalisation, les $\Delta$QTc, $\Delta$Tamp et $\Delta$TpTe calculées sont les valeurs absolues des différences, et pas les variations en pourcentage.

e. effectuer une analyse en composantes principales sur les valeurs $\Delta$QTc, $\Delta$Tamp et $\Delta$TpTe afin de générer trois nouvelles variables quantitatives PC1, PC2 et PC3

L'analyse en composantes principales (ACP ou PCA en anglais) est une méthode permettant de transformer des variables corrélées en nouvelles variables décorrélées les unes des autres.

Dans un mode de réalisation particulier, on effectue une ACP pour transformer les trois variables $\Delta$QTc, $\Delta$Tamp et $\Delta$TpTe en trois nouvelles variables décorrélées PC1, PC2 et PC3.

L'analyse en ACP est une méthode connue dans l'art, qui peut être réalisée notamment à l'aide de tout logiciel connu dans l'art.

L'algorithme utilisable dans les méthodes décrites ci-dessus est la formule permettant d'obtenir la variable PC1 à partir des variables $\Delta$QTc, $\Delta$Tamp et $\Delta$TpTe.

Parmi les trois variables PC1, PC2 et PC3 obtenues, la variable PC1 considérée est la variable qui est la mieux corrélée à chacun des $\Delta$QTc, $\Delta$Tamp et $\Delta$TpTe. Cela signifie que le coefficient de corrélation que l'on peut calculer pour PC1 avec chacun des $\Delta$QTc, $\Delta$Tamp et $\Delta$TpTe est supérieur (en valeur absolue) au coefficient de corrélation que l'on calcule pour les autres composantes principales PC2 et PC3 pour le $\Delta$QTc, $\Delta$Tamp ou $\Delta$TpTe en question. Le tableau 1 des exemples montre bien que la valeur absolue du coefficient de corrélation de PC1 est supérieure à la valeur absolue du coefficient de corrélation des PC2 et PC3 calculé pour chacun des $\Delta$QTc, $\Delta$Tamp et $\Delta$TpTe.

Si l'on a calculé $\Delta$Tamp selon la formule indiquée ci-dessus de telle sorte que $\Delta$Tamp soit positive, alors PC1 est corrélée de la même manière (coefficient de corrélation présentant le même signe) avec les $\Delta$QTc, $\Delta$Tamp et $\Delta$TpTe. Si l'on a $\Delta$TAmp négatif, PC1 est corrélée dans le même sens pour $\Delta$QTc et $\Delta$TpTe, et dans l'autre sens pour $\Delta$Tamp. Le tableau 1 des exemples montre une PC1 est corrélée négativement avec $\Delta$QTc, $\Delta$Tamp et $\Delta$TpTe, alors que PC2 est corrélée négativement avec $\Delta$QTc et positivement avec $\Delta$Tamp et $\Delta$TpTe. PC3 est corrélée négativement avec $\Delta$Tamp et positivement avec $\Delta$TpTe et $\Delta$QTc. On remarque aussi que les coefficients de corrélation de chaque valeur « $\Delta$ » sont plus élevés avec PC1 qu'avec l'une ou l'autre nouvelle variable.

Les éléments ci-dessus décrivent donc une méthode pour la génération d'un algorithme permettant de générer une valeur quantitative associée à l'apparition de signe ECG déterminant une inhibition d'IKr, c'est-à-dire un risque d'épisode de torsade de pointes à QT long, comprenant les étapes mentionnées ci-dessus.

Est ainsi décrite une méthode (*in vitro* ou *ex vivo*) pour la génération d'un algorithme permettant de générer une valeur quantitative associée à l'apparition de signe ECG déterminant une inhibition d'IKr, c'est-à-dire un risque d'épisode de torsade de pointes à QT long, comprenant les étapes

i) obtenir les valeurs $\Delta$QTc, $\Delta$TAmp et $\Delta$TpTe correspondant à la différence, en pourcentage, entre les valeurs QTc, TAmp et TpTe obtenues avant et après administration d'un médicament allongeant le QT, de préférence en inhibant le canal IKr, pour chacun des patients d'une cohorte

ii) effectuer une analyse en composantes principales sur ces valeurs $\Delta$QTc, $\Delta$TAmp et $\Delta$TpTe afin de générer trois nouvelles variables quantitatives PC1, PC2 et PC3.

L'algorithme ainsi généré est la formule permettant d'obtenir la variable PC1 à partir des variables $\Delta$QTc, $\Delta$Tamp et $\Delta$TpTe. La valeur PC1 est celle décrite ci-dessus.

Ainsi que vu plus haut, les méthodes décrites peuvent être utilisées pour déterminer le risque individuel d'un patient de présenter un trouble de la repolarisation ventriculaire en cas d'administration d'une substance connue pour présenter un effet torsadogénique, et que ladite composition administrée au patient est le sotalol.

On envisage donc une méthode pour déterminer le risque individuel d'un patient de présenter un trouble de la repolarisation ventriculaire en cas d'administration d'une substance connue pour présenter un effet torsadogénique, caractérisée en ce que l'on applique la méthode décrite ci-dessus audit patient, ladite composition administrée audit patient étant le sotalol, selon les conditions ci-dessus. L'analyse du risque individuel est effectuée selon les éléments mentionnés plus haut.

Les méthodes décrites peuvent également être utilisées pour déterminer si une substance d'intérêt (molécule test, ou médicament test) présente un caractère torsadogénique. Dans ce mode de réalisation, les méthodes selon l'invention sont répétées sur une cohorte de patient contenant nombre de patients supérieur ou égal à 2. Les différents résultats obtenus pour chaque patient sont ensuite analysés selon les informations fournies ci-dessus.

Ainsi que vu plus haut, on préfère lorsque la méthode est répétée, sur chaque patient, avec la substance d'intérêt et au moins une autre composition choisie dans le groupe constitué d'un placébo et d'un contrôle positif, et notamment lorsque le contrôle positif est choisi parmi le sotalol et la moxifloxacine, de préférence lorsqu'au moins un contrôle positif est le sotalol. Dans ce cas, il est préférable de l'utiliser à une dose de 80 mg en prise unique.

La divulgation se rapporte également à un dispositif pour la mise en oeuvre des méthodes décrites ci-dessus, ou un microprocesseur comprenant un algorithme permettant la mise en oeuvre des méthodes décrites ci-dessus. De fait, les méthodes décrites ci-dessus peuvent être mises en oeuvre par ordinateur.

## BREVES DESCRIPTION DES FIGURES

D'autres caractéristiques et avantages de l'invention ressortiront à la lecture de la description détaillée qui suit, en référence aux figures annexées, qui illustrent :

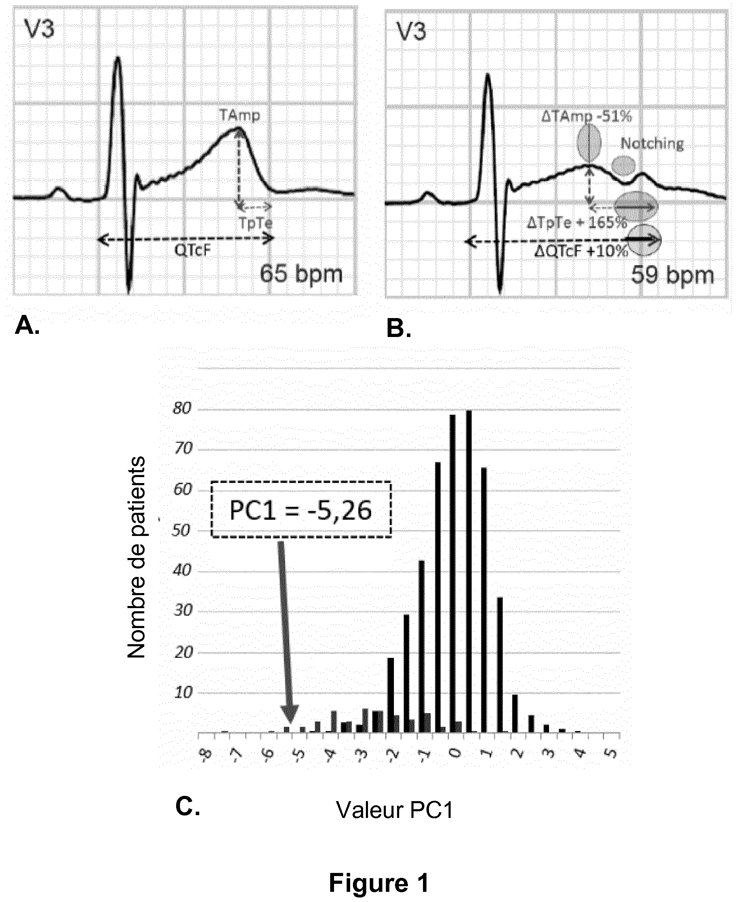

La figure 1 montre un tracé d'électrocardiogramme représentatif, chez un patient, avant (1.A) et trois heures après prise de sotalol (1.B), ainsi que la valeur PC1 obtenue pour ce patient, représentée par rapport à la distribution des valeurs PC1 dans la population (1.C). On observe le double pic de l'onde T (notch), et que la valeur PC1 est parmi les plus éloignées de 0.

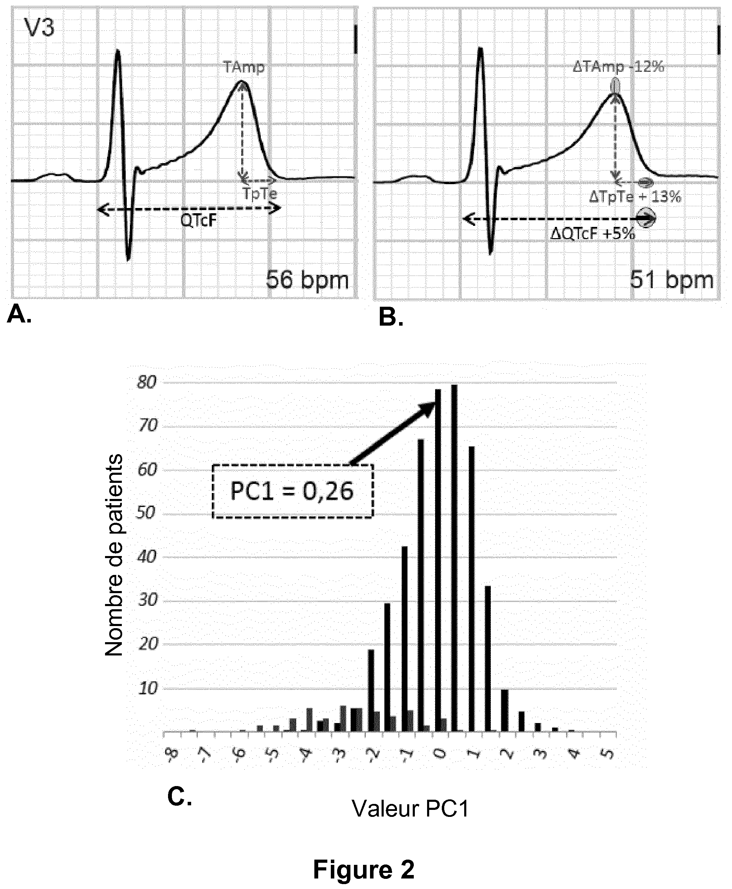

La figure 2 montre un tracé d'électrocardiogramme représentatif, chez un autre patient, avant (1.A) et trois heures après prise de sotalol (1.B), ainsi que la valeur PC1 obtenue pour cet autre patient, représentée par rapport à la distribution des valeurs PC1 dans la population (1.C). Ce patient présente une inhibition faible d'IKR induite par sotalol. La valeur PC1 calculée est donc proche de 0.

## EXEMPLES

### Exemple comparatif 1 -Génération d'un algorithme

*Population / Cohorte de patients*

La cohorte de patients était celle de l'étude GENEREPOL, et consistait en 995 volontaires en bonne santé, âgés de 18 à 60 d'origine européenne ou nord-africaine. L'objectif de cette étude était d'identifier des prédispositions génétiques liées à l'inhibition du canal IKr après administration de 80mg sotalol, avec un indice de masse corporelle entre 19 et 29kg/m$^2$, le consentement éclairé ayant été reçu.

Certains critères d'exclusion ont été utilisés : grossesse, asthme, fréquence cardiaque inférieure à 50 bpm, ECG (électrocardiogramme) anormal ou QRS>100ms, pression sanguine systolique <100 mmHg, antécédents de bloc atrio-ventriculaire ou phénomène de Raynaud, maladie chronique connue telle qu'insuffisance cardiaque ou rénale nécessitant un traitement chronique, médicament prolongeant le QT ou tout traitement chronique, excepté les pilules contraceptives les antalgiques et vitamines, antécédents familiaux ou personnels de syndrome du QT long congénital, d'arythmie, mort subite, ou QTc (méthode de Fridericia, QTcF)>450ms.

*Protocole de mesure de l'ECG*

Les volontaires ont été hospitalisés à 8h00 pendant une durée d'environ 6 heures après un jeûne nocturne.

Un cathéter intraveineux a été disposé pour effectuer les prises de sang. Puis on a enregistré l'ECG des patients en utilisant le dispositif Cardioplug (Cardionics Inc®, Bruxelles, Belgique) connecté à un ordinateur portable. Les enregistrements d'ECG de base (effectués chacun en triple, pendant 10 secondes) ont été obtenus après repos des patients pendant 10 minutes couchés sur le dos. On a ensuite donné une dose orale unique de 80 mg de sotalol aux patients et poursuivi le suivi ECG. Trois heures après prise de sotalol (H10), on a de nouveau enregistré l'ECG (triplicatas) après que les sujets sont restés couchés sur le dos pendant 10 minutes et avant le déjeuner.

À H3, un échantillon sanguin a été prélevé pour déterminer la concentration plasmatique de sotalol. Les participants ont été libérés 5 à 6 heures après prise de sotalol après avoir vérifié que QTcF était inférieur au QTcF de base + 40 ms.

*Analyse des ECG*

Dans la mesure où l'augmentation du QT par le sotalol représente l'équivalent de la forme iatrogène congénitale du syndrome du QT long LQT2, on a vérifié l'apparition des différences classiques entre le tracé de base et le tracé trois heures après prise de sotalol sur l'ECG.

Les QTcF, TpTe, TAmp et la présence d'encoches (double pic) dans l'onde T (notch) ont été quantifiés. La correction de Fridericia (QTcF) a été utilisée pour corriger le QT par rapport à la fréquence cardiaque, en accord avec les directives ICH E14.

L'analyse des données d'ECG a été effectuée avec le logiciel CARDIABASE (Groupe Banook, Nancy, France).

L'analyse quantitative des QTcF, TpTe and TAmp a été effectuée par deux investigateurs entraînés, afin de limiter la variabilité inter-évaluateur.

TpTe a été mesurée par la méthode de tangente, en triplicata, sur un battement représentatif (représentation moyennée d'un ECG de 10 secondes) sur les dérivations V3, V4 et V5. En cas de double pic de l'onde T (notch), le pic pris en considération a été le premier pic, même si l'amplitude de l'onde T était moins importante pour ce pic. La valeur moyenne obtenue a été retenue. En cas d'impossibilité de mesure de TpTe sur V3, V4 ou V5, on a, à la place, effectué la mesure sur V2 (pour V3), ou V6 (pour V4/V5).

Afin de mesurer, TAmp, on s'est positionné sur l'endroit de l'amplitude maximale du premier pic de l'onde T sur un battement représentatif (représentation moyennée d'un ECG de 10 secondes) sur les dérivations DII, V2, et V3. On a calculé et retenu la moyenne des valeurs TAmp issues de ces trois dérivations, et mesurées sur trois différents ECG de 10 secondes (triplicatas). S'il n'était pas possible de mesurer une TAmp sur une de ces dérivations (notamment en raison d'une faible valeur (<0.1mV)), on a utilisé la dérivation V4.

QTcF a été mesurée par la méthode de la tangente sur la dérivation DII pour trois battements consécutifs et la valeur moyenne d'une évaluation en triplicata a été retenue.

On a pu vérifier que les mesures restaient cohérentes et reproductibles entre les évaluateurs.

Pour ces paramètres, on a mesuré la différence entre la valeur obtenue à H3 et la valeur de base. On a exprimé cette différence en pourcentage par rapport à la valeur de base, et on a effectué les calculs de telle sorte que la valeur calculée soit positive.

On a donc effectué les calculs suivants :

$$\Delta QTcF\ (\%) = (QTcF\ à\ H3 - QTcF\ basal)\ /\ (QTcF\ basal)\ x\ 100$$

$$\Delta TpTe\ (\%) = (TpTe\ à\ H3 - TpTe\ basal)\ /\ (TpTe\ basal)\ x\ 100$$

$$\Delta TAmp\ (\%) = (TAmp\ basal - TAmp\ à\ H3)\ /\ (TAmp\ basal)\ x\ 100$$

Pour évaluer les doubles pics (notchs), tous les ECG ont été évalués indépendamment par deux investigateurs. Les patients ont été qualifiés de *notchers* ou *non-notchers,* en cas d'accord entre les deux investigateurs. En cas de discordance dans l'évaluation, les sujets n'ont pas été inclus dans ces groupes (seuls 10 sujets dans la cohorte d'évaluation et 8 sujets dans la cohorte de réplication n'ont pu être ainsi classifiés).

*Analyse en composantes principales*

Une analyse en composantes principales a été effectuée sur les valeurs calculées $\Delta QTcF$, $\Delta TpTe$ et $\Delta TAmp$, ce qui a permis de générer trois marqueurs alternatifs non corrélés, prenant mieux en compte l'information portée par les trois valeurs initiales corrélées. Les analyses statistiques ont été effectuées avec le logiciel R (https://www.r-project.org/).

*Résultats*

Changements dans l'électrocardiogramme induits par le Sotalol

Les changements observés dans l'électrocardiogramme trois heures après administration du sotalol suggèrent une inhibition du canal IKr, avec des résultats et observations similaires dans les cohortes d'exploration et de réplication, quels que soient les paramètres qualitatifs ou quantitatifs évalués.

En accord avec des études précédentes, le sotalol induit également des changements absolus et relatifs dans la durée du QTcF (21.4±14ms et 5.5±3.5% respectivement).

Des changements typiques dans la forme de l'onde T ont également été observés, avec une augmentation du TpTe (14.2±15.6 vs 15.9±20.5%, p=ns (non significatif)) et une diminution de TAmp (13.6±15.7 vs 12.8±15.3%, p=ns) respectivement entre les cohortes d'exploration et de réplication.

On a toutefois observé une grande variabilité inter-sujets dans tous les changements observés. Les variations de TpTe, TAmp et QTcF entre H3 et le niveau basal présentent une autocorrélation dans la même proportion entre les deux cohortes.

Enfin, 40 (8 %) ou 51 (10 %) sujets présentaient un double pic (notch) dans les deux cohortes. Il s'agissait presque exclusivement de femmes, et ces patients avaient un $\Delta TpTe$, $\Delta QTcF$ et $\Delta TAmp$ supérieur à ceux observés pour les non-notchers (patients sans double pic).

Analyse en composantes principales

L'analyse en composantes principales a permis de générer trois nouvelles valeurs quantitatives, liées aux modifications de repolarisation ventriculaire induites par le sotalol.

Le premier composant (PC1) expliquant environ 65-67 % de la variance phénotypique reflète les modifications typiques après inhibition du canal IKr, c'est-à-dire l'augmentation de TpTe, QTcF et la diminution de TAmp (Tableau 1).

On a observé une diminution significative de la valeur PC1 ($p<10^{-4}$) chez les patients présentant un double pic (*notchers*) par rapport aux patients ne présentant pas ce double pic (non-notchers) dans l'une ou l'autre des cohortes d'exploration (-2,5±1,5 vs 0,3±1,1) ou de réplication (-2,3±1,8 vs 0,3±1,1).

La valeur PC1 est donc une valeur alternative quantitative de l'inhibition de IKr.

La distribution des valeurs PC1 est montrée aux Figures 1 et 2 avec l'exemple d'un patient présentant une inhibition IKr extrême, avec double pic, et une valeur PC1 négative extrême (Figure 1), et un patient présentant une inhibition faible de IKr (Figure 2).

Les autres composantes principales (PC2 et PC3) expliquaient respectivement environ 19-21% et 14% de la variance phénotypique.

Tableau 1. Corrélations (r) entre ΔTAmp, ΔTpTe, ΔQTcF et les composantes principales dans les cohortes d'exploration (n=489, partie supérieure du tableau) ou de réplication (n=495, partie inférieure du tableau)

| Corrélation(r) | ΔTAmp | ΔTpTe | ΔQTcF | PC1 | PC2 | PC3 |
|---|---|---|---|---|---|---|
| ΔTAmp | 1 | 0,59 | 0,40 | -0,83 | 0,36 | -0,43 |
| ΔTpTe | 0,58 | 1 | 0,45 | -0,85 | 0,23 | 0,47 |
| ΔQTcF | 0,47 | 0,46 | 1 | -0,73 | -0,68 | -0,06 |
| PC1 | -0,84 | -0,84 | -0,78 | 1 | 0 | 0 |
| PC2 | 0,28 | 0,3 | -,063 | 0 | 1 | 0 |
| PC3 | -0,46 | 0,45 | 0,01 | 0 | 0 | 1 |

On observe bien que PC1 est corrélée dans le même sens avec ΔTAmp, ΔTpTe, et ΔQTcF (corrélée négativement dans cet exemple), ce qui n'est pas le cas pour PC2 ou PC3.

On observe également que la valeur absolue des coefficients de corrélation de PC1 avec ΔTAmp, ΔTpTe, et ΔQTcF est supérieure à celle des PC2 ou PC3 avec ces valeurs.

*Conclusion*

Comme attendu, une augmentation des TpTe, QTcF et une diminution de Tamp ont été observés entre le niveau basal et trois heures après prise de sotalol.

La nouvelle valeur PC1 obtenue par analyse en composantes principales de ΔQTcF, ΔTpTe et ΔTAmp explique l'essentiel de la variance totale des données « Δ » (ΔQTcF, ΔTpTe et ΔTAmp) et est ainsi associée au mécanisme commun d'augmentation de QTcF et TpTe, associé à la diminution de TAmpl, et est un marqueur quantitatif et intégratif de l'inhibition IKr induite par médicament.

**Exemple 2** - **Application de l'algorithme pour identifier une interaction médicamenteuse à sur-risque d'inhibition d'IKr**

*Population*

L'étude a été réalisée sur 615 femmes en bonne santé, de type européen ou nord-africain, entre 18 et 60 ans, avec un QTcF < 450ms, et aucune maladie ou chronique connue, ou traitement chronique, excepté contraception, et sans antécédents familiaux de syndrome du QT long congénital, arythmie, ou mort subite.

*Mesure et calcul des différentes données QTcF, Tamp et TpTe*

Les QTcF, TAmp et TpTe ont été mesurés selon le même protocole que pour l'exemple 1, avant et trois heures après administration d'une dose orale unique de sotalol à 80 mg.

On a aussi calculé les ΔQTcF, ΔTpTe et ΔTAmp.

*Analyse en composantes principales*

Une analyse en composantes principales a été effectuée sur les $\Delta$QTcF, $\Delta$TpTe et $\Delta$TAmp (logiciel XLStat, Addinsoft®) afin de générer trois nouvelles valeurs décorrélées.

*Résultats*

La variable PC1, corrélée de la même façon avec $\Delta$QTcF, $\Delta$TpTe et $\Delta$TAmp (c'est-à-dire qui est le plus corrélée et dans le même sens avec chacune de ces variables), explique 63 % de la variance phénotypique observée (caractérisée par l'augmentation de QTcF et TpTe et la diminution de TAmp).

On observe également que la valeur absolue de PC1 est significativement supérieure pour les femmes ayant une contraception sous drospirénone comparé au groupe de femmes n'ayant pas de contraception (respectivement 0,56 [-0,13; 1,5] et 0,09[-0,58; 1,1], p<0,05). Dans cet exemple, PC1 issu de l'analyse d'un sous-groupe exclusif de femmes est corrélé positivement à $\Delta$QTcF (r=0.71), $\Delta$TpTe (r=0.85) et $\Delta$TAmp (r=0.83) et les valeurs plus positives de PC1 sont liées à une inhibition d'IKr plus importante.

De même, les femmes sous drospirénone présentaient plus de doubles pics trois heures après prise de sotalol (25,8%) que les femmes ne prenant pas de contraceptif oral (13,4%).

Aucune différence n'a été observée pour ce qui est de la concentration plasmatique de sotalol dans ces deux groupes.

Ces résultats sont cohérents avec les propriétés pharmacologiques de la drospirénone, qui a un potentiel androgénique inférieur à d'autres pilules, le caractère protecteur de la testostérone étant connu.

## Conclusion générale

L'exemple 1 montre bien que l'application d'un algorithme permet de déterminer la réponse individuelle d'un patient au sotalol, et la sensibilité de ce patient à l'inhibition du canal IKr par les médicaments (et donc au risque de torsade de pointes).

L'exemple 2 montre bien que la méthode ici décrite (utilisation d'un algorithme basé sur l'analyse en composantes principales de $\Delta$QTcF, $\Delta$TpTe et $\Delta$TAmp) permet d'identifier, pour une substance donnée (ici la drospirénone), le caractère d'induction d'allongement du QT et d'inhibition du canal IKr.

## Revendications

1. Méthode *ex vivo* mise en oeuvre par ordinateur pour déterminer le risque d'induction d'un effet torsadogénique après administration d'une composition chez un patient comprenant les étapes :

    I. Combiner, les valeurs $\Delta$QTc, $\Delta$Tamp et $\Delta$TpTe correspondant à la différence, en pourcentage ou valeur absolue, entre les valeurs QTc, Tamp et TpTe obtenues avant et après administration de ladite composition, par la formule a1 $\times$ $\Delta$TAmp(%) + a2 $\times$ $\Delta$QTcf(%) + a3 x $\Delta$TpTe(%) + b, avec -0,05 $\leq$ a1 $\leq$ -0,03 ; -0,2 $\leq$ a2 $\leq$ -0,1 ; -0,04 $\leq$ a3 $\leq$ -0,02 ; 1,5 $\leq$ b $\leq$ 2
    II. Obtenir un résultat final de cette combinaison ;
    III. Comparer la valeur de ce résultat final à une valeur de référence.

2. Méthode selon la revendication 1, **caractérisée en ce que** le résultat final obtenu à l'étape II est obtenu par application de la formule : a1 $\times$ $\Delta$TAmp(%) + a2 $\times$ $\Delta$QTcf(%) + a3 $\times$ $\Delta$TpTe(%) + b, avec a1 = -0,038 $\pm$ 0,002; a2 = -0,15 $\pm$ 0,001 ; a3 = -0,031 $\pm$ 0,002; b = 1,79 $\pm$ 0,05, correspondant à la valeur PC1.

3. Méthode selon l'une des revendications 1 ou 2, **caractérisée en ce qu'**elle est utilisée pour déterminer le risque individuel d'un patient de présenter un trouble de la repolarisation ventriculaire en cas d'administration d'une substance connue pour présenter un effet torsadogénique, et que ladite composition administrée au patient est le sotalol.

4. Méthode selon l'une des revendications 1 ou 2, **caractérisée en ce que** ladite composition est une substance d'intérêt et que cette méthode est utilisée pour déterminer si la substance d'intérêt présente, en tant que telle, un caractère torsadogénique, ladite méthode selon la revendication 1 étant répétée sur une cohorte de patient contenant nombre de patients supérieur ou égal à 2.

5. Méthode selon la revendication 4, **caractérisée en ce que** la méthode est répétée, sur chaque patient, avec la substance d'intérêt et au moins une autre composition choisie dans le groupe constitué d'un placébo et d'un contrôle positif.

6. Méthode selon la revendication 5, **caractérisée en ce que** le contrôle positif est choisi parmi le sotalol et la moxifloxacine.

7. Méthode selon la revendication 6, **caractérisée en ce que** le contrôle positif est le sotalol, utilisé à 80 mg en prise unique, et que ladite substance d'intérêt présente un caractère plus torsadogénique que le sotalol si la moyenne des résultats finaux obtenus pour les patients de la cohorte après administration de la substance d'intérêt est inférieure à la moyenne des résultats finaux obtenus pour les patients de la cohorte après administration du sotalol.

8. Méthode selon l'une des revendications 1 à 7, **caractérisée en ce que** TAmp a été mesuré sur les dérivations D2, V2, V3.

9. Méthode selon l'une des revendications 1 à 8, **caractérisée en ce que** TpTe a été mesuré sur les dérivations V2, V3, V4.

10. Méthode selon l'une des revendications 1 à 9, **caractérisée en ce que** QTcF a été mesuré sur la dérivation D2.

11. Méthode selon l'une des revendications 1 à 10, **caractérisée en ce qu'**au moins une valeur TAmp, TpTe ou QTcF a été mesurée sur un complexe moyenné.

## Patentansprüche

1. Computerimplementierte Ex-vivo-Verfahren zur Bestimmung des Risikos der Induktion eines torsadogenen Effekts nach Verabreichung einer Zusammensetzung an einen Patienten, die die folgenden Schritte umfasst:

I. Kombinieren der $\Delta$QTc, $\Delta$Tamp und $\Delta$TpTe Werte, die der Differenz zwischen den QTc-, Tamp- und TpTe-Werten, die vor und nach der Verabreichung der Zusammensetzung erhalten wurden, in Prozent oder als absoluter Wert entsprechen, durch die Formel a1 x $\Delta$TAmp(%) + a2 x $\Delta$QTcf( %) + a3 x $\Delta$TpTe(%) + b, wobei $-0{,}05 \leq a1 \leq -0{,}03$; $-0{,}2 \leq a2 \leq -0{,}1$; $-0{,}04 \leq a3 \leq -0{,}02$; $1{,}5 \leq b \leq 2$
II. Erhalten eines Endergebnisses dieser Kombination;
III. Vergleich des Werts dieses Endergebnisses mit einem Referenzwert.

2. Verfahren nach Anspruch 1, **dadurch gekennzeichnet, dass** das in Schritt II erhaltene Endergebnis durch Anwenden der Formel a1 x $\Delta$TAmp(%) + a2 x $\Delta$QTcf(%) + a3 x $\Delta$TpTe(%) + b erhalten wird, wobei a1 = $-0{,}038 \pm 0{,}002$; a2 = $-0{,}15 \pm 0{,}001$; a3 = $-0{,}031 \pm 0{,}002$; b = $1{,}79 \pm 0{,}05$, entsprechend dem Wert PC1.

3. Verfahren nach einem der Ansprüche 1 oder 2, **dadurch gekennzeichnet, dass** es zur Bestimmung des individuellen Risikos eines Patienten, eine ventrikuläre Repolarisationsstörung nach Verabreichung einer Substanz, von der bekannt ist, dass sie eine torsadogene Wirkung aufweist, zu entwickeln, verwendet wird, und dass die dem Patienten verabreichte Zusammensetzung Sotalol ist.

4. Verfahren nach einem der Ansprüche 1 oder 2, **dadurch gekennzeichnet, dass** die Zusammensetzung eine interessierende Substanz ist und dass dieses Verfahren verwendet wird, um zu bestimmen, ob die interessierende Substanz als solche einen torsadogenen Charakter aufweist, wobei das Verfahren nach Anspruch 1 an einer Patientengruppe wiederholt wird, die eine Anzahl von Patienten enthält, die größer oder gleich 2 ist.

5. Verfahren nach Anspruch 4, **dadurch gekennzeichnet, dass** das Verfahren bei jedem Patienten mit der interessierenden Substanz und mindestens einer anderen Zusammensetzung, die aus der Gruppe ausgewählt ist, die aus einem Placebo und einer positiven Kontrolle besteht, wiederholt wird.

6. Verfahren nach Anspruch 5, **dadurch gekennzeichnet, dass** die positive Kontrolle aus Sotalol und Moxifloxacin ausgewählt wird.

7. Verfahren nach Anspruch 6, **dadurch gekennzeichnet, dass** die positive Kontrolle Sotalol ist, das in einer Einzeldosis von 80 mg verwendet wird, und dass die interessierende Substanz einen stärker torsadogenen Charakter als Sotalol aufweist, wenn der Mittelwert der Endergebnisse, die für die Patienten in der Kohorte nach Verabreichung der interessierenden Substanz erhalten werden, niedriger ist als der Mittelwert der Endergebnisse, die für die Patienten in der Kohorte nach Verabreichung von Sotalol erhalten werden.

8. Verfahren nach einem der Ansprüche 1 bis 7, **dadurch gekennzeichnet, dass** TAmp an den Ableitungen D2, V2, V3 gemessen wurde.

9. Verfahren nach einem der Ansprüche 1 bis 8, **dadurch gekennzeichnet, dass** TpTe an den Ableitungen V2, V3, V4 gemessen wurde.

10. Verfahren nach einem der Ansprüche 1 bis 9, **dadurch gekennzeichnet, dass** QTcF an der Ableitung D2 gemessen wurde.

11. Verfahren nach einem der Ansprüche 1 bis 10, **dadurch gekennzeichnet, dass** mindestens ein TAmp-, TpTe- oder QTcF-Wert an einem gemittelten Komplex gemessen wurde.

**Claims**

1. A computer-implemented ex *vivo* method for determining the risk of induction of a torsadogenic effect after administration of a composition to a patient comprising the steps:

   I. Combining the $\Delta$QTc, $\Delta$Tamp and $\Delta$TpTe values corresponding to the difference, in percentage or absolute value, between the QTc, Tamp and TpTe values obtained before and after administration of said composition, by the formula a1 x $\Delta$TAmp(%) + a2 x $\Delta$QTcf(%) + a3 x $\Delta$TpTe(%) + b, with $-0.05 \leq a1 \leq -0.03$ ; $-0.2 \leq a2 \leq -0.1$; $-0.04 \leq a3 \leq -0.02$; $1.5 \leq b \leq 2$
   II. Obtaining a final result of this combination;
   III. Comparing the value of this final result with a reference value.

2. Method according to claim 1, **characterized in that** the final result obtained in step II is obtained by applying the formula: a1 x $\Delta$TAmp(%) + a2 x $\Delta$QTcf(%) + a3 x $\Delta$TpTe(%) + b, where a1 = $-0.038 \pm 0.002$; a2 = $-0.15 \pm 0.001$; a3 = $-0.031 \pm 0.002$; b = $1.79 \pm 0.05$, corresponding to the value PC1.

3. Method according to one of claims 1 or 2, **characterized in that** it is used to determine a patient's individual risk of exhibiting a ventricular repolarization disorder upon administration of a substance known to exhibit a torsadogenic effect, and that said composition administered to the patient is sotalol.

4. Method according to one of claims 1 or 2, **characterized in that** said composition is a substance of interest and that this method is used to determine whether the substance of interest as such exhibits a torsadogenic character, said method according to claim 1 being repeated on a patient cohort containing a number of patients greater than or equal to 2.

5. Method according to claim 4, **characterized in that** the method is repeated, on each patient, with the substance of interest and at least one other composition chosen from the group consisting of a placebo and a positive control.

6. Method according to claim 5, **characterized in that** the positive control is chosen from sotalol and moxifloxacin.

7. Method according to claim 6, **characterized in that** the positive control is sotalol, used at 80 mg as a single dose, and that said substance of interest has a more torsadogenic character than sotalol if the mean of the final results obtained for the patients in the cohort after administration of the substance of interest is lower than the mean of the final results obtained for the patients in the cohort after administration of sotalol.

8. Method according to one of claims 1 to 7, **characterized in that** TAmp was measured on D2, V2, V3 leads.

9. Method according to one of claims 1 to 8, **characterized in that** TpTe was measured on V2, V3, V4 leads.

10. Method according to one of claims 1 to 9, **characterized in that** QTcF has been measured on D2 lead.

11. Method according to one of claims 1 to 10, **characterized in that** at least one TAmp, TpTe or QTcF value has been measured on an averaged complex.

**Figure 1**

Figure 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2008188761 A

**Littérature non-brevet citée dans la description**

- **SEKARSKI et al.** *Paediatrica,* 2008, vol. 19 (4
- **COUDERC et al.** *Heart Rhythm,* Juillet 2011, vol. 8 (7), 1036-43
- **RECANATINI et al.** *Med Res Rev.,* Mars 2005, vol. 25 (2), 133-66
- **CLARK et al.** *J Chem Inf Model.,* Novembre 2009, vol. 49 (11), 2617-26
- **BOTTINO et al.** *Prog Biophys Mol Biol.,* Janvier 2006, vol. 90 (1-3), 414-43
- **SUZUKI et al.** *Prog Biophys Mol Biol.,* Septembre 2008, vol. 98 (1), 52-60